# EUROPÄISCHE PATENTSCHRIFT

(11) **EP 2 695 588 B1**
(45) Veröffentlichungstag und Bekanntmachung des Hinweises auf die Patenterteilung: **11.10.2017**
(21) Anmeldenummer: 13003917.5
(22) Anmeldetag: 06.08.2013
(51) Int. Cl.: A61F 5/00, A61C 7/08

(54) **Medizinische Vorrichtung zur Bekämpfung von Übergewicht oder Fettsucht beim Menschen**
Medical device for controlling excess weight or obesity in humans
Dispositif médical pour lutter contre la surcharge pondérale ou l'obésité chez l'homme

(30) Priorität: 09.08.2012 DE 102012015839
(43) Veröffentlichungstag der Anmeldung: 12.02.2014
(60) Teilanmeldung: 17001367.6
(73) Patentinhaber: von Seck, Peter, 65185 Wiesbaden (DE)
(72) Erfinder: von Seck, Peter, 65185 Wiesbaden (DE)
(74) Vertreter: Henseler, Daniela

(56) Entgegenhaltungen:
- NL-C- 2 005 541
- US-A- 5 899 691
- US-A1- 2003 059 737
- US-A1- 2009 035 729

## Beschreibung

Die Erfindung betrifft eine Vorrichtung zur Bekämpfung von Übergewicht oder Fettsucht beim Menschen.

Aus GB 2 433 203 A ist eine gattungsgemäße medizinische Vorrichtung zur Bekämpfung von Übergewicht oder Fettsucht beim Menschen bekannt. Danach ist vorgesehen eine Vorrichtung zur Bisshebung, die ein kastenförmiges Gebilde über mindestens einen Teil der Kauflächenzähne befestigt anordnet. Als Kaufläche entsteht dann eine Oberfläche, die deckelartig die Kauflächenbereiche der Zähne überdeckt.

Aus US 5,899,691 A ist ein Kautrainer aus einer weichen Masse bekannt, um das Kiefergelenk und dessen Muskeln zu stärken. Die auf der Kaufläche angeordnete Kaumasse ist an der Kaufläche fixiert, um deren Position beim Kauen beizubehalten.

Aus US 4,738,259 ist eine Gewichtskontrollvorrichtung bekannt, die den natürlichen Kauvorgang blockiert. Linguale Kragen sind vorgesehen, die eine Bewegung von Speisen zu und von dem Kaubereich verhindern.

Wie DE 10 2005 041 093 A1 beschreibt, ist Fettleibigkeit ein aktuelles Zivilisationsproblem, welches in den meisten Fällen auf falsche Essgewohnheiten, Bewegungsmangel etc. zurückzuführen ist. Dabei ist bekannt, dass gerade solche Essgewohnheiten, die zu Fettleibigkeit führen, auf undiszipliniertem Essverhalten seitens der betreffenden Patienten beruhen. Aus diesem Grund werden Diäten von diesen Patienten häufig nicht durchgehalten, oder die Patienten fallen nach Beendigung der Diät in ihre alten Essgewohnheiten zurück.

Es ist daher zur Lösung dieser Problematik bekannt, Bereiche des Verdauungstraktes, insbesondere den Magen, durch einen chirurgischen Eingriff zu verkleinern und eine Kurzschlussverbindung mit dem Dünndarm zu schaffen. Der Patient erfährt hierdurch bereits mit Aufnahme relativ kleiner Nahrungsportionen ein Völlegefühl, was ihn letztlich von weiterer Nahrungsaufnahme abhält. Für eine möglichst gering belastende Verkleinerung können Clips endoskopisch an entsprechenden Positionen einer Organinnenseite platziert, ein Band eingefädelt und durch zusammenziehen der beiden freien Enden des Bandes ein Einschnüren des Organs bewirkt werden. Die natürliche Funktion dieser so veränderten Organe bleibt nicht erhalten, so dass neben der invasiven Belastung des Patienten auch Organschäden die Folge sind.

Aufgabe der Erfindung ist es daher, eine Vorrichtung zur Bekämpfung von Übergewicht oder Fettsucht beim Menschen zu schaffen, die falsches Essfehlverhalten korrigiert und dabei in einer den Patienten gering beeinträchtigenden Weise einsetzbar ist.

Diese Aufgabe wird gelöst durch eine medizinische Vorrichtung zur Bekämpfung von Übergewicht oder Fettsucht beim Menschen durch Erreichen eines schnelleren Sättigungsgefühls, nach Anspruch 1. Hierdurch wird eine Vorrichtung zur Bekämpfung von Übergewicht oder Fettsucht beim Menschen geschaffen, die eine Verlangsamung des Kauvorganges bewirkt. Die Verlangsamung des Kauvorganges führt zu einer erhöhten Kauleistung. Die erfindungsgemäße okklusale Erhöhung beeinflusst den mechanischen Kauvorgang durch eine Veränderung der Beschaffenheit der Oberflächengestaltung der Kaufläche, d.h. der okklusalen Fläche, eines Kauflächenzahnes. Der Vollkontakt der natürlichen okklusalen Fläche eines Kauflächenzahnes wird verkleinert. Zur Aufnahme von Nahrung muss deshalb die Kauleistung erhöht werden.

Kauen ist die mechanische Aufbereitung der Nahrung durch okklusalen Quetschdruck. Die Zähne sind dabei die eigentlichen Werkzeuge des Kauapparates. Zwischen ihren Kronen entstehen die Kaukräfte. Wird nun erfindungsgemäß die Kaufläche verkleinert, sind mehr Kaubewegungen erforderlich, um die Nahrung mechanisch aufzubereiten. Erreicht wird eine Veränderung der Zeit der Nahrungsaufnahme. Die Essenszeit für eine Mahlzeit wird z. B. verlängert. Der Patient wird zum langsamen Essen gezwungen, da statt der üblichen 5 bis 10 Kaubewegungen mehr Kaubewegungen, beispielsweise 20 bis 40 Kaubewegungen, erforderlich sind, um die gleiche Nahrungsmenge zu kauen. Langsames Essen wird so trainiert.

Erfindungsgemäß wird ferner erreicht, dass die Nahrungsaufnahme einer Mahlzeit sich derart verlängert, dass das von Hormonen gesteuerte Sättigungsgefühl, das etwa 15 bis 30 Minuten verzögert dem Essen folgt, bereits während der Nahrungsaufnahme einer Mahlzeit eintreten kann. Der Patient ist folglich satt, obwohl die aufgenommene Nahrungsmenge geringer ist. Das erfindungsgemäß erzwungene Langsamessen verhindert Übergewicht bzw. führt zur nachhaltigen Reduktion von Übergewicht.

Die Schienen können leicht eingesetzt werden und aus unterschiedlichen Materialien hergestellt sein. Die Schienen können temporär nur während des Essens eingesetzt werden und sind deshalb vorzugsweise herausnehmbar ausgebildet. Die Schienen statten jeweils den ersten und zweiten Prämolar und den ersten Molar des Ober- und Unterkiefers mit einer okklusalen Erhöhung aus. Die Okklusionsbewegung des Kiefers unter Zahn- bzw. Kauflächenführung wird dadurch nicht nachteilig beeinflusst. Die Schlussbissstellung ergibt eine hinreichend stabile Lage einer mechanischen Okklusion.

Weitere Ausgestaltungen und Vorteile der Erfindung sind der nachfolgenden Beschreibung und den Unteransprüchen zu entnehmen.

Die Erfindung wird nachstehend anhand der in den beigefügten Abbildungen dargestellten Ausführungsbeispiele näher erläutert.
Fig. 1 zeigt schematisch einen Schnitt eines ersten Molaren mit einer erfindungsgemäßen Schiene,
Fig. 2 zeigt schematisch einen Schnitt einer okklusalen Kontaktform zweier Kauflächenzähne mit einer erfindungsgemäßen Schiene am Oberkiefer und Unterkiefer,
Fig. 3 zeigt schematisch eine Seitenansicht einer okklusalen Kontaktform,
Fig. 4 zeigt das Zahnschema des permanenten Gebisses mit Zahlenanordnung nach dem alten deutschen System.

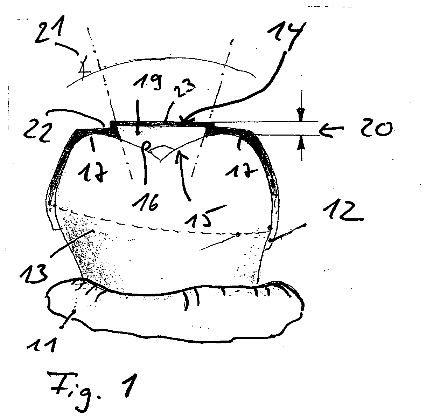

Wie Fig. 1 bis Fig. 3 zeigen, betrifft die Erfindung eine medizinische Vorrichtung zur Bekämpfung von Übergewicht oder Fettsucht beim Menschen durch Erreichen eines schnelleren Sättigungsgefühls. Die Vorrichtung ist dazu ausgebildet als mindestens eine der mit dem menschlichen Oberkiefer 10 und/oder Unterkiefer 11 verbindbaren Schienen 12, die mindestens einen Teil der Kauflächenzähne 13 mit einer okklusalen Erhöhung 14 zur Verkleinerung der Kaufläche ausstattet. Die Schiene 12 ist eine Aufbissschiene, die an Zahnkronen der Kauflächenzähne 13 angepasst ist, wobei die Zahnkrone der obere Anteil eines Zahnes ist, der aus dem Zahnfleisch herausragt.

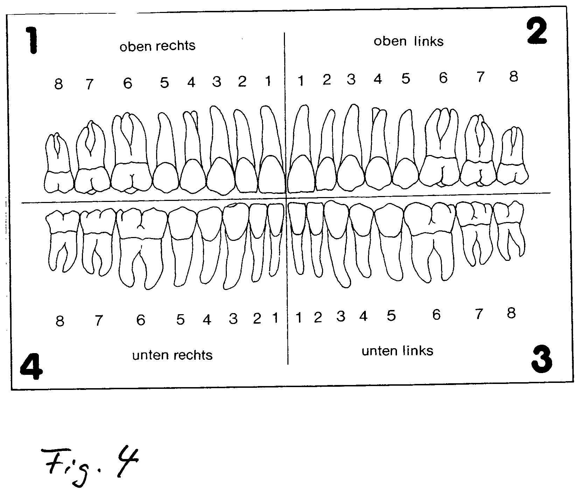

Die nach ihrer Funktion genannten Kauflächenzähne 13 sind die Prämolare und Molare, jeweils rechts und links im Ober- und Unterkiefer, die man auch unter dem Oberbegriff Seitenzähne zusammenfasst. Ausweislich Fig. 4, das alte deutsche System, kennzeichnet die Zahl 4 den jeweils ersten Prämolar, die Zahl 5 den jeweils zweiten Prämolar und die Zahlen 6, 7, 8 den ersten, zweiten und dritten Molar des Oberkiefers und Unterkiefers.

Die Kauflächenzähne 13 besitzen ein Kaurelief, für das sich der Begriff Kaufläche 15 eingebürgert hat, obwohl es keine Fläche, sondern ein Gebilde aus Höckern, Graten, Leisten und Gräben ist. Die Funktion der Kauflächenzähne ist, den mit den Frontzähnen aufgenommenen Speisebrocken einzuspeicheln und zu zerkleinern. Durch den Kontakt der oberen und unteren Kauflächenzähne 13 (Seitenzähne) ineinander fügen sich bei der natürlichen Okklusion harmonisch ineinander unter Bildung des Kauzentrums.

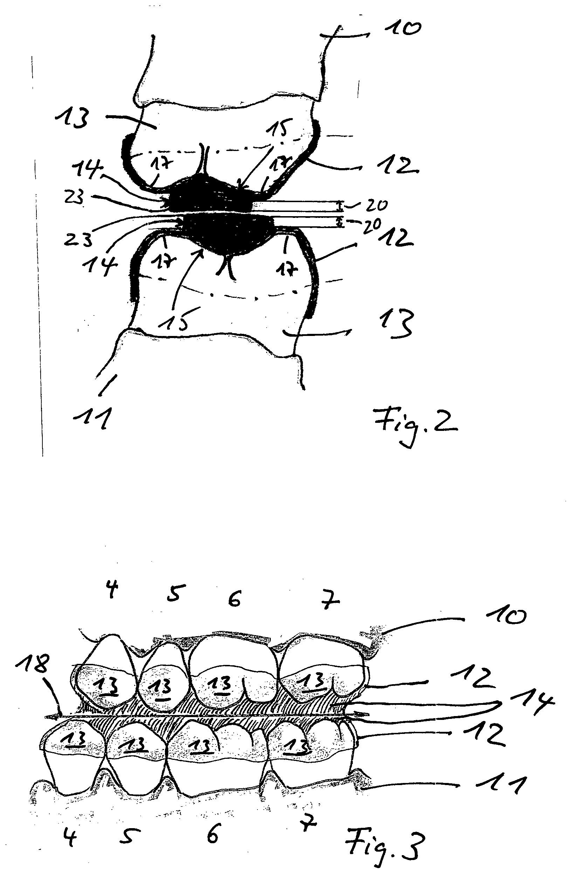

Durch die Schienen 12 wird dieses Kauzentrum verkleinert, indem die Kaufläche 15 eine okklusale Erhöhung 14 entlang mindestens eines Teils der Kauflächenzähne 13 erhält. Diese Erhöhung 14 kann so ausgebildet sein, dass die Gräben 16 mindestens teilweise oder ganz aufgefüllt werden, wodurch eine quasi plane Kaufläche als Kauebene 18 von der okklusalen Erhöhung 14 bereitgestellt wird, wie dies in Fig. 1 und Fig. 3 dargestellt ist. Die Kaufläche 15 ist dadurch bereits,reduziert. Zudem sind die Höckerspitzen 17 eines Kauflächenzahnes 13 überbaut durch die okklusale Erhöhung 14, wie dies ebenfalls Fig. 1 zeigt. Eine Bisserhöhung 20 wird hierdurch verknüpft mit einem Überspannen des Kaureliefs.

Die okklusale Erhöhung 14 bildet die Bisserhöhung 20 aus an einem Stegabschnitt 23, der als ein vorstehender Abschnitt der Schiene 12 ausgebildet ist. Die Stegbreite des Stegabschnitts 23 ist geringer als die Breite der Kaufläche 15 über die Höckerspitzen 17. Besonders bevorzugt besitzt der Stegabschnitt 23 eine Breite, die den jeweiligen Graben 16 eines Kauflächenzahnes 13 im wesentlichen vorzugsweise mittig überdeckt. Über die Einstellung der Breite des Stegabschnitts 23 zwischen den Höckerspitzen 17 eines Kauflächenzahnes 13 kann die Höhe der Kauflächenreduzierung patientenbezogen gewählt werden. Die vorstehende Ausführungen gelten in gleicher Weise für einen Ober- und einen Unterkiefer, wie Figur 2 zeigt.

Die okklusale Erhöhung 14 stellt beispielsweise eine Bisserhöhung im Bereich von 0,5 bis 2 mm pro Kiefer 10, 11 ein. Durch eine wählbare Ausgestaltung der okklusalen Erhöhung 14 in Höhe und Breite kann die Kaufläche 15 um beispielsweise 10 bis 50% reduziert werden. Mindestens eine der Schienen 12 stellt eine okklusale Erhöhung 14 im Bereich der ersten und zweiten Prämolare (4, 5 nach Fig. 4) und des ersten Molars (6 nach Fig. 4) des Ober- und/oder Unterkiefers 10, 11 ein. Gemäß Fig. 4 erstreckt sich eine okklusale Erhöhung 14 auch über den zweiten Molar (7 nach Fig. 4). Die Schienen 12 können aus Kunststoff, Metall oder einem Keramikwerkstoff bestehen, wobei diese Materialien zahnfarben sein können. Diese Materialien können tiefgezogen, vergossen oder gefräst zum Einsatz kommen. Auch gemischt, d.h., Metallkauflächen auf einer Kunststoffschiene sind ausbildbar. Die Stärke und Beschaffenheit der Schienen 12 sind dabei einstellbar. Die Stärke der Schiene 12 liegt vorzugsweise außerhalb der Erhöhung 14 im Bereich von 0,3 bis 0,5 mm.

Die okklusale Erhöhung 14 kann ferner mit einem weichelastischen Material 19 (vgl. Fig. 1) unterfüttert sein. Das weichelastische Material ist insbesondere Silicon.

Die Schienen 12 sind vorzugsweise herausnehmbar für den Patienten einsetzbar, so dass die Schienen 12 nur zeitweilig getragen werden können, insbesondere begrenzt auf den Zeitraum der Nahrungsaufnahme. Daher sind Bedenken jeglicher Art über Kieferveränderungen oder Gelenkschmerzen zu vernachlässigen. Die Schienen 12 können friktionsgetragen, klammergetragen oder als Modellguss getragen sein.

Die okklusale Erhöhung 14 kann für einen Speichelabfluss mit einer Art Drainage oder Rinne 22 versehen sein. Wie Fig. 1 zeigt, ist die Erhöhung 14 dazu beispielsweise seitlich abgewinkelt, mit einem Winkel 21.

Schließlich können drucksensitive Bauteile in die Erhöhung 14 eingebaut sein für diagnostische oder therapeutische Zwecke im Bereich des Okklusionsfeldes.

Weiterhin können die Schienen mit einem Transponder, insbesondere einem passiven Transponder, ausgestattet sein, der die Identifizierung der Schienen ermöglicht.

## Patentansprüche

1. Medizinische Vorrichtung zur Bekämpfung von Übergewicht oder Fettsucht beim Menschen durch Erreichen eines schnelleren Sättigungsgefühls, umfassend eine mit dem menschlichen Oberkiefer (10) verbindbare Schiene (12) und eine mit dem menschlichen Unterkiefer verbindbare Schiene (12), die jeweils an die Zahnkronen angepasst sind und zur Bisserhöhung (20) mindestens einen Teil der okklusalen Fläche der Kauflächenzähne (13) überdecken, wobei die Schiene (12) des Ober- und Unterkiefers (10, 11) mindestens einen Teil der Kauflächenzähne (13) mit einer okklusalen, als ein vorstehender Stegabschnitt (23) der Schiene ausgebildeten Erhöhung (14) zur Verkleinerung der Kaufläche (15) ausstattet, die die Höckerspitzen (17) eines Kauflächenzahnes (13) überbaut zum Verknüpfen der Bisserhöhung (20) mit einem Überspannen des Kaureliefs des überdeckten Kauflächenzahnes (13), und die Schiene die okklusale Erhöhung (14) im Bereich der ersten und zweiten Prämolare (4, 5) und des ersten Molars (6) des Ober- und Unterkiefers (10, 11) einstellt, so dass die Schlussbissstellung eine hinreichend stabile Lage einer mechanischen Okklusion mit verkleinertem Kauzentrum ergibt.

2. Medizinische Vorrichtung nach Anspruch 1, **dadurch gekennzeichnet, dass** die okklusale Erhöhung (14) eine Bisserhöhung im Bereich von 0,5 bis 2 mm pro Kiefer (10, 11) einstellt.

3. Medizinische Vorrichtung nach Anspruch 1 oder 2, **dadurch gekennzeichnet, dass** die okklusale Erhöhung (14) die Kaufläche (15) um 10 bis 50% reduziert.

4. Medizinische Vorrichtung nach einem der Ansprüche 1 bis 3, **dadurch gekennzeichnet, dass** sich die okklusale Erhöhung (14) auch über den zweiten Molar (7) erstreckt.

5. Medizinische Vorrichtung nach einem der Ansprüche 1 bis 4, **dadurch gekennzeichnet, dass** die Schiene (12) aus Kunststoff, Metall oder einem Keramikwerkstoff besteht.

6. Medizinische Vorrichtung nach einem der Ansprüche 1 bis 5, **dadurch gekennzeichnet, dass** die okklusale Erhöhung (14) mit einem weichelastischen Material unterfüttert ist.

7. Medizinische Vorrichtung nach Anspruch 6, **dadurch gekennzeichnet, dass** das weichelastische Material Silicon ist.

8. Medizinische Vorrichtung nach einem der Ansprüche 1 bis 7, **dadurch gekennzeichnet, dass** die Schiene (12) friktionsgetragen, klammergetragen oder als Modellguss getragen ist.

## Claims

1. Medical device for combating overweight or obesity in humans by achieving a more rapid feeling of satiety, comprising a splint (12) being connectable to the human maxilla (10) and a splint being connectable to the human mandible, which are adapted to the tooth crowns, respectively, and cover at least some of the occlusal surface of the masticatory teeth (13) for bite elevation (20),
wherein the splint (12) of the maxilla and the mandible (10, 11) equips at least some of the masticatory teeth (13) with an occlusal elevation (14) being designed as a protruding base section (23) of the splint for reducing the size of the masticatory surface (15), which occlusal elevation overlies the cusp tips (17) of a masticatory tooth (13) in order to link the bite elevation (20) to a spanning of the masticatory relief of the covered masticatory tooth (13)
and the splint sets the occlusal elevation (14) in the area of the first and second premolars (4, 5) and of the first molar (6) of the maxilla and mandible (10, 11), such that the final bite setting provides a sufficiently stable position of a mechanical occlusion with a mastication centre being reduced in size.

2. Medical device according to Claim 1, **characterized in that** the occlusal elevation (14) sets a bite elevation in the range of 0.5 to 2 mm per jaw (10, 11).

3. Medical device according to Claim 1 or 2, **characterized in that** the occlusal elevation (14) reduces the masticatory surface (15) by 10 to 50%.

4. Medical device according to one of Claims 1 to 3, **characterized in that** the occlusal elevation (14) also extends over the second molar (7).

5. Medical device according to one of Claims 1 to 4, **characterized in that** the splint (12) is made of plastic, metal or a ceramic material.

6. Medical device according to one of Claims 1 to 5, **characterized in that** the occlusal elevation (14) is lined underneath with a soft elastic material.

7. Medical device according to Claim 6, **characterized in that** the soft elastic material is silicone.

8. Medical device according to one of Claims 1 to 7, **characterized in that** the splint (12) is supported by friction, supported by clasps or supported as model casting.

## Revendications

1. Dispositif médical pour lutter contre la surcharge pondérale ou l'obésité chez l'homme par obtention d'une sensation de satiété plus rapide, comprenant une gouttière (12) pouvant être reliée à la mâchoire supérieure humaine (10) et une gouttière (12) pouvant être reliée à la mâchoire inférieure humaine, qui sont adaptées respectivement aux couronnes dentaires et recouvrent au moins une partie de la surface occlusale des dents à surface de mastication (13) pour la surélévation occlusale (20), dans lequel la gouttière (12) de la mâchoire supérieure et inférieure (10, 11) dote au moins une partie des dents à surface de mastication (13) d'une élévation (14) occlusale réalisée en tant que section de moulure en saillie (23) de la gouttière pour la réduction de la surface de mastication (15), qui couvre les pointes de cuspides (17) d'une dent à surface de mastication (13) pour associer la surélévation occlusale (20) à un enjambement du relief de mastication de la dent à surface de mastication (13) recouverte, et la gouttière règle l'élévation occlusale (14) au niveau des première et deuxième prémolaires (4, 5) et de la première molaire (6) de la mâchoire supérieure et inférieure (10, 11), de sorte que la position de morsure finale donne une position suffisamment stable d'une occlusion mécanique avec centre de mastication réduit.

2. Dispositif médical selon la revendication 1, **caractérisé en ce que** l'élévation occlusale (14) règle une surélévation occlusale de l'ordre de 0,5 à 2 mm par mâchoire (10, 11).

3. Dispositif médical selon la revendication 1 ou 2, **caractérisé en ce que** l'élévation occlusale (14) réduit la surface de mastication (15) de 10 à 50 %.

4. Dispositif médical selon l'une quelconque des revendications 1 à 3, **caractérisé en ce que** l'élévation occlusale (14) s'étend aussi sur la deuxième molaire (7).

5. Dispositif médical selon l'une quelconque des revendications 1 à 4, **caractérisé en ce que** la gouttière (12) est en plastique, métal ou en un matériau céramique.

6. Dispositif médical selon l'une quelconque des revendications 1 à 5, **caractérisé en ce que** l'élévation occlusale (14) est calée avec un matériau élastique souple.

7. Dispositif médical selon la revendication 6, **caractérisé en ce que** le matériau élastique souple est de la silicone.

8. Dispositif médical selon l'une quelconque des revendications 1 à 7, **caractérisé en ce que** la gouttière (12) est portée par friction, par clip ou comme prothèse.
